# EUROPEAN PATENT APPLICATION

(11) **EP 1 435 230 A2**
(43) Date of publication of application: **07.07.2004**
(21) Application number: 03258040.9
(22) Date of filing: 19.12.2003
(51) Int. Cl.: A61K 7/48, A61K 7/42

(54) **Sunscreen compositions**

(30) Priority: 30.12.2002 US 331297
(71) Applicant: NEUTROGENA CORPORATION, Los Angeles California 90045-5544 (US)
(72) Inventor: Singleton, Laura C., Los Angeles CA 90043 (US)
(74) Representative: Fisher, Adrian John

(57) **Abstract**

The present invention relates to a composition containing (i) a lipophilic sunscreen, (ii) a copolymer of sodium acryloyldimethyltaurate and one or more acryls, and (iii) an oil-absorbant.

## Description

### FIELD OF THE INVENTION

The present invention relates to a sunscreen composition.

### BACKGROUND OF THE INVENTION

The prolonged exposure to ultra-violet (UV) radiation, such as from the sun, can lead to the formation of light dermatoses and erythemas, as well as increase the risk of skin cancers, such as melanoma. UV radiation can also accelerate skin aging, such as loss of skin elasticity and wrinkling. Radiation with wavelengths in the UV-A range (from about 320 to 400 nm) and the UV-B range (from about 280 to about 320 nm) can cause such skin damage, and, thus, sunscreen compositions should preferably comprise both UV-A and UV-B sunscreens. Many of these sunscreens, however, are lipohphilic, and thus leave an oily-feel on the skin of the user.

The present invention relates to a sunscreen composition that combines the use of emulsifying-gelling agents and oil-absorbing agents to reduce the oil feel of the sunscreen agents.

### SUMMARY OF THE INVENTION

The present invention relates to a composition containing (i) a lipophilic sunscreen, (ii) a copolymer of sodium acryloyldimethyltaurate and one or more acryls, and (iii) an oil-absorbant.

Other features and advantages of the present invention will be apparent from the detailed description of the invention and from the claims.

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. Unless otherwise indicated, a percentage refers to a percentage by weight (i.e., %(W/W)).

### Definitions

What is meant by a "product" is a product in finished packaged form. In one embodiment, the package is a container such as a plastic, metal or glass tube or jar containing the composition. The product may further contain additional packaging such as a plastic or cardboard box for storing such container. In one embodiment, the product contains instructions directing the user to apply the composition to the skin to provide protection from ultraviolet light (e.g., UVA and/or UVB light from the sun). Such instructions may be printed on the container, label insert, or on any additional packaging.

What is meant by "promoting" is promoting, advertising, or marketing. Examples of promoting include, but are not limited to, written, visual, or verbal statements made on the product or in stores, magazines, newspaper, radio, television, internet, and the like. Examples of such statements include, but are not limited to, "sunscreen," "sunblock," "sunprotection," "ultraviolet protection," and "ultraviolet block."

As used herein, "topically applying" means directly laying on or spreading on outer skin, nails, or hair, e.g., by use of the hands or an applicator such as a wipe, roller, or spray.

As used herein, "cosmetically-acceptable" means that the ingredients which the term describes are suitable for use in contact with tissues (e.g., the skin or hair) without undue toxicity, incompatibility, instability, irritation, allergic response, and the like.

As used herein, "safe and effective amount" means an amount of the sunscreen or of the composition sufficient to protect the user's skin from ultraviolet light (e.g., UVA and/or UVB light), but low enough to avoid serious side effects. The safe and effective amount of the sunscreens or composition will vary with the area being treated, the age and skin type of the end user, the duration and nature of the treatment, the specific ingredient or composition employed, the particular cosmetically-acceptable carrier utilized, and like factors.

### Sunscreens

The composition of the present invention contains one or more lipophilic sunscreens. What is meant by a "sunscreen" is a compound that absorbs, reflects, or scatters radiation in the UV range. Examples of lipophilic sunscreens include, but are not limited to, octocrylene, homosalate, octinoxate, octisalate, avobenzone, oxybenzone, benzophenone-1, benzophenone-2, benzophenone-8, benzophenone-12, ethyl dihydroxypropyl PABA, gyceryl PABA, menthyl antranilate, ethylhexyl dimethyl PABA, methylbenzylidene camphor, isopropyl dibenzoyl methane, and etocrylene.

In one embodiment, the composition comprises a safe and effective amount of one or more lipophilic sunscreens. In one embodiment, the composition comprises from about 0.1 to about 50 percent, by weight, of one or more lipophilic sunscreens. In one embodiment, the composition comprises at least 10 percent by weight of one or more lipophilic sunscreens.

In one embodiment, the composition of the present invention may also contain non-lipophilic sunscreens such as titanium dioxide, zinc oxide, phenylbenzimidazole sulfonic acid, benzophenone-4, TEA salicylate, PABA, and DEA Methoxycinnamate

### Copolymer of Sodium Acryloyldimethyltaurate

The composition of the present invention contains a copolymer of sodium acryloyldimethyltaurate and one or more acryls. Examples of acryls include, but are not limited to, acrylic acids, acrylates, acrylamides, methylacrylic acids, methylacrylates, and methylacrylamide and salts thereof such as sodium acrylates, hydroxyethyl acrylate, and acrylamide. Examples of such copolymer include, but are not limited to, Hydroxyethyl acrylate/ Acryloyldimethyltaurate (available as Simulgel® NS from Seppic Inc., Fairfield, NJ), Sodium acrylate/Acryloyldimethyltaurate (available as Simulgel® EG from Seppic Inc.), and Acrylamide/Acryloyldimethyltaurate (available as Simulgel® 600 from Seppic Inc.)

In one embodiment, the composition comprises from about 0.1 to about 10 percent, by weight, of said copolymer. In one embodiment, the composition comprises at least 1 percent, by weight, of said copolymer.

### Oil-absorbents

The composition of the present invention comprises an oil-absorbent. What is meant by an oil-absorbant is a solid compound that can attract oil (e.g, imbibe the oil or attach the oil to its surface). Examples of oil-absorbing agents include, but are not limited to silica (e.g., spherical silicas, porous silicas, and fumed silica powders), Polymethyl Methacrylate, Dextrins, Cyclodextrins, Sericites, Molecular Sievs-Zeolites, Nylons (e.g., Nylon 6 or 12), Sodium Calcium Aluminosilicate, Boron Nitrade, Calcium Aluminum Borosilicate (Luxsil), Lauroyl Lysine, PTFE, Bismuth Oxycloride, Aluminium Starch Octenylsuccinate, Calcium Starch Octenylsuccinate, Sodium Starch Octenylsuccinate, Starches (e.g. Solanum Tuberosum (Potato),Oryza Sativa (Rice),Zea Mays(Corn)), Titanium Dioxide, Zinc Oxide, Talc, Mica, Hydroxyapatite, Magnesium Aluminometasilicate, Magnesium Aluminum Silicate, Magnesium Carbonate, Calcium Carbonate, Barium Sulphate, Tricalcium Phosphate, Silk Powder, Kaolin, Bentonite, Hectorite, Crosslinked PMMA, and Polysilicones (e.g., polysilicone-11).

### Additional Cosmetically Active Agents

In one embodiment, the topical composition further contains another cosmetically active agent in addition to the sunscreen. What is meant by a "cosmetically active agent" is a compound (e.g., a synthetic compound or a compound isolated from a natural source) that has a cosmetic or therapeutic effect on the skin, hair, or nails, including, but not limiting to, lightening agents, darkening agents such as self-tanning agents, anti-acne agents, shine control agents, anti-microbial agents, anti-inflammatory agents, anti-mycotic agents, anti-parasite agents, external analgesics, antioxidants, keratolytic agents, detergents/surfactants, moisturizers, nutrients, vitamins, energy enhancers, anti-perspiration agents, astringents, deodorants, hair removers, firming agents, anti-callous agents, and agents for hair, nail, and/or skin conditioning.

In one embodiment, the agent is selected from, but not limited to, the group consisting of hydroxy acids, benzoyl peroxide, D-panthenol, carotenoids, free radical scavengers, spin traps, retinoids such as retinol and retinyl palmitate, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, peptides containing copper such as Cu:Gly-His-Lys, coenzyme Q10, amino acids such a proline, vitamins, lactobionic acid, acetyl-coenzyme A, niacin, riboflavin, thiamin, ribose, electron transporters such as NADH and FADH2, and other botanical extracts such as aloe vera, and derivatives and mixtures thereof. The cosmetically active agent will typically be present in the composition of the invention in an amount of from about 0.001% to about 20% by weight of the composition, e.g., about 0.005% to about 10% such as about 0.01% to about 5%.

Examples of vitamins include, but are not limited to, vitamin A, vitamin Bs such as vitamin B3, vitamin B5, and vitamin B12, vitamin C, vitamin K, and vitamin E and derivatives thereof.

Examples of hydroxy acids include, but are not limited, to glycolic acid, lactic acid, malic acid, salicylic acid, citric acid, and tartaric acid. See, e.g., European Patent Application No. 273,202.

Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like.

Examples of such natural extracts include grape seed, green tea, pine bark, and propolis.

### Other Materials

Various other materials may also be present in the compositions useful in the subject invention. These include humectants, proteins and polypeptides, chelating agents (e.g., EDTA), and preservatives (e.g., parabens), and pH adjusting agents. In addition, the topical compositions useful herein can contain conventional cosmetic adjuvants, such as dyes, opacifiers (e.g., titanium dioxide), pigments, and fragrances.

### Mineral Water

The compositions of the present invention may be prepared using a mineral water, for example mineral water that has been naturally mineralized such as Evian® Mineral Water (Evian, France). In one embodiment, the mineral water has a mineralization of at least about 200 mg/L (e.g., from about 300 mg/L to about 1000 mg/L). In one embodiment, the mineral water contains at least about 10 mg/L of calcium and/or at least about 5 mg/L of magnesium.

The composition and formulations containing such compositions of the present invention may be prepared using methodology that is well known by an artisan of ordinary skill.

### Example 1: Sunscreen Compositions

The following is a description of the manufacture of a composition according to the present invention (Comp 1) having an SPF of 45. Other compositions may be made in a similar manner, and a suggested range of the amounts of each ingredient is also indicated in Table 1.

**Table 1**

| | | | Comp. 1 | Range |
|---|---|---|---|---|
| | CTFA NAME | SUPPLIER | (%W/W) | (%W/W) |
| 1 | WATER | | 43.66 | |
| 2 | HOMOSALATE | Eusolex HMS Rona EM Industries, Hawthorne, NY | 15 | 0.1-15 |
| 3 | ETHYLHEXYL METHOXYCINNAMATE | Escalol 557 ISP , 7Bound Brook, NJ | 7.5 | 0.1-7.5 |
| 4 | BENZOPHENONE -3 | Uvinul M-40 USP/ BASF Co. North Mount Olive, NJ | 6 | 0.1-6 |
| 5 | ETHYLHEXYL SALICYLATE | Neoheliopan, Type OS Harmaan & Reimer Rosemont, IL | 5 | 0.1- 5 |
| 6 | SILICA | Spheron L-1500 Presperse Inc., Plainfield, NJ | 3.5 | 0.1-20 |
| 7 | HYDROXYETHYL ACRYLATE & SODIUM ACRYLOYLDIMETHYLTAURATE COPOLYMER & SQUALANE & POLYSORBATE 60 | Simulgel NS Seppic Inc. Fairfield, NJ | 3 | 0.1-7 |
| 8 | AVOBENZONE | Parsol 1789 Roche Parsippany, NJ | 2 | 0.1-3 |
| 9 | ADIPIC ACID&DIETHYLENE GLYCOL&GLYCERYN CROSSPOLYMER | Loxorez100 Inolex, Philadelphia, PA | 2 | 0.1-10 |
| 10 | PVP-HEXADECENE COPOLYMER | Ganex V216 ISP Bound Brook, NJ | 2 | 0.1-10 |
| 11 | DIMETHICONE, 200 fluid, 50 cst. | D.C.200 Fluid Dow Corning Corp. Midland, MI | 1.5 | 0.1-10 |
| 12 | GLYCERYL STEARATE & PEG-100 STEARATE | Arlacel 165 ICI, Wilmington, Delaware | 1.25 | 0.1-10 |
| 13 | GLYCERIN | Emry 916 Cognis, Corp. Ambler, PA | 1 | 0.1-10 |
| 14 | DIMETHICONE & TRIMETHYLSILOXY SILICATE | D.C. Fluif 593 Dow Corning Corp. Midland, MI | 1 | 0.1-10 |
| 15 | BENZYL ALCOHOL | Benzyl Alcohol Charckit Co. Darien, CT | 1 | 0.01-1 |
| 16 | OAT KERNAL EXTRACT | Oat Extract Resources of Nature Inc. Hazlet, NJ | 1 | 0.01-10 |
| 17 | ISOPROPYLPARABEN & ISOBUTYLPARABEN & BUTYLPARABEN | Liquapar Oil ISP-Sutton Corp., Chatham NJ | 0.8 | 0.1-1 |
| 18 | PHENOXYETHANOL | Emeresssence 1160 Cognis, Corp. Ambler, PA | 0.6 | 0.1-1 |
| 19 | TOCOPHERYL ACETATE | Vitamin E Acetate Roche, Parsippany, NJ | 0.5 | 0.1-10 |
| 20 | BISABOLOL | Bisabolol BASF Co., North Mount Olive, NJ | 0.5 | 0.1-10 |
| 21 | CETYL DIMETHICONE | Abilwax 9801 Goldschmidt, Hopewell, Virginia | 0.5 | 0.1-10 |
| 22 | XANTHAN GUM | Keltrol CG CP Kelco San Diego, CA | 0.3 | 0.01-2 |
| 23 | DIPOTASSIUM GLYCYRRHETINATE | Dipotassium Glycyrrhetinate Barnet, Englewood Cliffs, NJ | 0.1 | 0.01-10 |
| 24 | TETRASODIUM EDTA | Hamp-ene 220 Soco-Lynch Nashua, NH | 0.1 | 0.01-1 |
| 25 | BHT | Naugard BHT Uniroyal Chemical Geismar LO | 0.07 | 0.01-1 |
| 26 | FRAGRANCE | | 0.1 | 0.01-5 |
| 27 | ASCORBYL PALMITATE | C-Palmitate Roche Parsippany, NJ | 0.01 | 0.01-10 |
| 28 | RETINYL PALMITATE | A-Palmitate Roche Parsippany, NJ | 0.01 | 0.01-10 |

In a primary container, the xanthan gum, dipotassium glycyrrihizinate, tetrasodium EDTA, and glycerin were added and mixed with the water until the gum completely hydrated ("Water Phase"). The Water Phase was then heated to 70-75°C. In a separate container, ingredients nos. 2-5, 8-12, 14, 17-21, 25, 27, and 28 were added heated to 70-75°C and mixed to assure that the sunscreens were completely dissolved ("Oil Phase"). The Oil Phase was then slowly added to the Water Phase and allowed to mix until an emulsion was formed. The emulsion was homogenized for one minute at 70% power using a homogenizer (Greerco 1L made by Kenics, Hudson, New Hampshire). The mixing was continued with high agitation while the ingredient no. 7 was being added. The mixing was continued until a homogenous emulsion was formed. The resulting emulsion was then cooled to 50-55°C, following which the silica powder was sprinkled into the emulsion. The cooling of the emulsion continued to 40-45°C, following which the benzyl alcohol, oat kernal extract, and fragrance were added to the emulsion. The missing continued until the emulsion had cooled to 30°C.

### Example 2: Assessment of Oil/Lipids Deposited on the Skin

To assess the levels of oil and lipids deposited on the skin by Comp. 1 of Example 1 and Coppertone Sport SPF 48 (Schering-Plough, Memphis, TN) which contains silica but not the copolymer of sodium acryloyldimethyltaurate), the volar forearms of five subjects were chosen as the test site. Four circular areas of 1-inch diameter were delineated on each of their volar forearms. Baseline measurements were performed in triplicates on each area using Sebumeter™ (Courage and Khazaka, Koln, Germany). Each test formulation was applied at 0.02 gram/cm² to two of the areas. After 15 and 30 minutes after application, the level of oil and lipids deposited on the skin was measured using Sebumeter™ in triplicates on one of the areas specified for the time point. The levels of oil/lipids deposited on the skin were determined from the mean value of the triplicate measurements using the equation: level of oil/lipids = mean (after) - mean (before).

The level of oil/lipids at 15 minutes after application were 254 mg/cm² for Comp. 1 and 284 mg/cm² for the Coppertone Sport SPF 48 (p value = 0.02) and at 30 minutes after application were 257 mg/cm² for Comp. 1 and 270 mg/cm² for the Coppertone Sport SPF 48 (p value = 0.107). Thus, the application of Comp. 1 resulted in the deposition of significantly less oil/sebum of the skin following application of the product as compared to Coppertone Sport SPF 48.

### Example 3: Assessment of Shine

The following experiment was conducted to compare the shine produced by Comp. 1 of Example 1 and Coppertone Sport SPF 48. Two subjects washed their face with Neutrogena Fresh Foaming Cleanser (Neutrogena Corporation, Los Angeles, CA) and allowed it to dry for ten minutes. A baseline digital image was taken using polarized light. Both products were dispensed in a quantity of 0.3 grams and each applied to a different side of the face, which were randomly selected. Digital polarized images were taken at 15, 30, and 60 minutes following applications. The luminosity of these images were then analyzed using PhotoShop Version 6.0 (Adobe Systems Incorporated, San Jose, California, USA). Table 2 shows the average change in luminosity from baseline for the two products.

**Table 2**

| **Time (Min)** | **Comp. 1** | **Coppertone** |
|---|---|---|
| 15 | 0.007 | 0.077 |
| 30 | -0.021 | 0.076 |
| 60 | 0.005 | 0.061 |

Thus, Coppertone Sport SPF 48 substantially increased the luminosity of the face following application as compared to Comp 1 which maintained the luminosity to about baseline levels.

### Example 4: Assessment of Shine

The following experiment was conducted to assess the affect of the inclusion of silica on levels of shine on the skin produced by the sunscreen formulations. Comp 1 of Example 1 ("Silica Product") was compared against the same formulation without the inclusion of silica ("Non-silica Product"). Ten subjects were recruited as panelists to evaluate the shine in a blinded manner. The back of hands was used as the test site. The formulations were first filled into syringes that were coded so as to not reveal the identity of the formulations. Both of the formulations were dispensed on the back of hands, one on each hand, in 0.1 ml aliquots. The subjects evenly spread the dispensed materials with the palm of the other hand. The subjects then evaluated the shine under a regular fluorescent lamp in order to select the shinier side. Eighty percent of the test subjects concluded that the Non-Silica Product produced a shinier appearance on their skin, while the remaining twenty percent could not distinguish between the two products.

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

## Claims

1. A composition comprising (i) a lipophilic sunscreen, (ii) a copolymer of sodium acryloyldimethyltaurate and one or more acryls, and (iii) an oil-absorbant.

2. A composition of claim 1, wherein said composition comprises at least 10 percent by weight of one or more lipophilic sunscreens.

3. A composition of claim 1, wherein said lipophilic sunscreen is selected from the group consisting of octocrylene, homosalate, octinoxate, octisalate, avobenzone, and oxybenzone.

4. A composition of claim 2, wherein at least one of said one or more lipophilic sunscreens is selected independently from the group consisting of octocrylene, homosalate, octinoxate, octisalate, avobenzone, and oxybenzone.

5. A composition of claim 1, wherein said oil-absorbant is selected from the group consisting of silica, aluminum starch octentylsuccinate, lauroyl lysine, boron nitrate, magnesium aluminometasilicate, polymethyl methacrylate, nylon, and talc.

6. A composition of claim 2, wherein said oil-absorbant is selected from the group consisting of silica, aluminum starch octentylsuccinate, lauroyl lysine, boron nitrate, magnesium aluminometasilicate, polymethyl methacrylate, nylon, and talc.

7. A composition of claim 3, wherein said oil-absorbant is selected from the group consisting of silica, aluminum starch octentylsuccinate, lauroyl lysine, boron nitrate, magnesium aluminometasilicate, polymethyl methacrylate, nylon, and talc.

8. A composition of claim 4, wherein said oil-absorbant is selected from the group consisting of silica, aluminum starch octentylsuccinate, lauroyl lysine, boron nitrate, magnesium aluminometasilicate, polymethyl methacrylate, nylon, and talc.

9. A composition of claim 1, wherein said copolymer is a copolymer of sodium acryloyldimethyltaurate and hydroxyethylacrylate, and said oil-absorbant is silica.

10. A composition of claim 2, wherein said copolymer is a copolymer of sodium acryloyldimethyltaurate and hydroxyethylacrylate, and said oil-absorbant is silica.

11. A sunscreen composition comprising (i) from about 0.1 to about 50 percent by weight of a lipophilic sunscreen, (ii) from about 0.1 to about 10 percent by weight of a copolymer of sodium acryloyldimethyltaurate and one or more acryls, and (iii) from about 0.1 to about 10 percent by weight of an oil-absorbant.

12. A composition of claim 14, wherein said composition comprises (i) at least about 10 percent by weight of one or more lipophilic sunscreens, (ii) at least about 1 percent by weight of one or more copolymers of sodium acryloyldimethyltaurate and one or more acryls, and (iii) at least about 1 percent by weight of one or more oil-absorbents.

13. A composition of claim 11, wherein said lipophilic sunscreen is selected from the group consisting of octocrylene, homosalate, octinoxate, octisalate, avobenzone, and oxybenzone.

14. A composition of claim 12, wherein at least one of said one or more lipophilic sunscreens is selected independently from the group consisting of octocrylene, homosalate, octinoxate, octisalate, avobenzone, and oxybenzone.

15. A composition of claim 11, wherein said oil-absorbant is selected from the group consisting of silica, aluminum starch octentylsuccinate, lauroyl lysine, boron nitrate, magnesium aluminometasilicate, polymethyl methacrylate, nylon, and talc.

16. A composition of claim 12, wherein said oil-absorbant is selected from the group consisting of silica, aluminum starch octentylsuccinate, lauroyl lysine, boron nitrate, magnesium aluminometasilicate, polymethyl methacrylate, nylon, and talc.

17. A composition of claim 13, wherein said oil-absorbant is selected from the group consisting of silica, aluminum starch octentylsuccinate, lauroyl lysine, boron nitrate, magnesium aluminometasilicate, polymethyl methacrylate, nylon, and talc.

18. A composition of claim 14, wherein said oil-absorbant is selected from the group consisting of silica, aluminum starch octentylsuccinate, lauroyl lysine, boron nitrate, magnesium aluminometasilicate, polymethyl methacrylate, nylon, and talc.

19. A composition of claim 11, wherein said copolymer is a copolymer of sodium acryloyldimethyltaurate and hydroxyethylacrylate, and said oil-absorbant is silica.

20. A composition of claim 12, wherein said copolymer is a copolymer of sodium acryloyldimethyltaurate and hydroxyethylacrylate, and said oil-absorbant is silica.
